# EUROPEAN PATENT SPECIFICATION

(11) **EP 2 344 673 B1**
(45) Date of publication and mention of the grant of the patent: **07.05.2014**
(21) Application number: 09812486.0
(22) Date of filing: 21.10.2009
(51) Int. Cl.: C12Q 1/68

(54) **DIAGNOSTIC TEST FOR STREPTOCOCCUS EQUI**
DIAGNOSTISCHER TEST FÜR STREPTOCOCCUS EQUI
ESSAI DIAGNOSTIQUE POUR STREPTOCOCCUS EQUI

(30) Priority: 21.10.2008 US 107108 P
(43) Date of publication of application: 20.07.2011
(73) Proprietor: Animal Health Trust, Kentford Newmarket Suffolk CB8 7UU (GB)
(72) Inventor: WALLER, Andrew, Stephen, Newmarket Suffolk CB8 7UU (GB); ROBINSON, Carl, Newmarket Suffolk CB8 7UU (GB); HEATHER, Zoe, Williamstown, VIC 3016 (AU)
(74) Representative: Kremer, Simon Mark
(86) International application number: PCT/GB2009/002518
(87) International publication number: WO 2010/046648

(56) References cited:
- ALBER J ET AL: "Multiplex polymerase chain reaction for identification and differentiation of Streptococcus equi subsp zooepidemicus and Streptococcus equi subsp equi" JOURNAL OF VETERINARY MEDICINE SERIES B, vol. 51, no. 10, December 2004 (2004-12), pages 455-458, XP002583373 ISSN: 0931-1793
- BAVERUD V ET AL: "Real-time PCR for detection and differentiation of Streptococcus equi subsp equi and Streptococcus equi subsp zooepidemicus" VETERINARY MICROBIOLOGY, vol. 124, no. 3-4, October 2007 (2007-10), pages 219-229, XP002583374 ISSN: 0378-1135
- GRONBAEK L MOLLER ET AL: "Evaluation of a nested PCR test and bacterial culture of swabs from the nasal passages and from abscesses in relation to diagnosis of Streptococcus equi infection (strangles)" EQUINE VETERINARY JOURNAL, vol. 38, no. 1, January 2006 (2006-01), pages 59-63, XP002583375 ISSN: 0425-1644
- PUSTERLA N ET AL: "Real-time polymerase chain reaction: A novel molecular diagnostic tool for equine infectious diseases" JOURNAL OF VETERINARY INTERNAL MEDICINE, vol. 20, no. 1, January 2006 (2006-01), pages 3-12, XP002583376 ISSN: 0891-6640
- HEATHER ZOE ET AL: "A novel streptococcal integrative conjugative element involved in iron acquisition" MOLECULAR MICROBIOLOGY, vol. 70, no. 5, December 2008 (2008-12), pages 1274-1292, XP002583377 ISSN: 0950-382X
- WEBB KATY ET AL: "Development of an unambiguous and discriminatory multilocus sequence typing scheme for the Streptococcus zooepidemicus group" MICROBIOLOGY (READING), vol. 154, no. Part 10, October 2008 (2008-10), pages 3016-3024, XP002583378 ISSN: 1350-0872 cited in the application

## Description

### Technical field

The present invention relates generally to methods and materials concerning diseases caused by *Streptococcus equi,* and in particular relating to the detection of this pathogen by amplification of nucleic acid.

### Background art

*Streptococcus* is a genus of spherical shaped Gram-positive bacteria. Clinically, individual species of *Streptococcus* are classified primarily based on their Lancefield serotyping - according to specific carbohydrates in the bacterial cell wall. These are named Lancefield groups A to T. However the pathogens in these different groups share many similarities at the genetic level. For example *Streptococcus equi* (which is in group C, and which is the causative agent of equine strangles) shares 80% genome identity with the human pathogen *S. pyogenes* (which is in group A, and which is the causative agent of many human conditions including strep throat, acute rheumatic fever, scarlet fever, acute glomerulonephritis and necrotizing fasciitis). Additionally the two organisms share many near identical toxins and virulence factors.

Streptococci are further characterised via their haemolytic properties. Alpha haemolysis is caused by a reduction of iron in haemoglobin giving it a greenish color on blood agar. Beta only haemolysis is complete rupture of red blood cells giving distinct, wide, clear areas around bacterial colonies on blood agar. Other streptococci are labeled as gamma haemolytic.

Strangles is a disease characterised by nasal discharge and fever, followed by abscessation of local lymph nodes. The swelling of the lymph nodes in the head and neck may, in severe cases, restrict the airway and it is this clinical feature that gave the disease 'strangles' its name. Morbidity rates of up to 100% are reported and mortality as a result of disseminated abscessation ('bastard strangles') may occur in 10% of cases (Timoney, 1993). Strangles is one of the most frequently diagnosed equine diseases worldwide. Recent outbreaks in Thoroughbreds have further highlighted the need for the development of improved diagnostic tests. In particular it is important to have highly sensitive and specific diagnostic tests that rapidly identify infected horses. These horses can then be isolated and the outbreak contained.

Approximately 10% of horses that recover from strangles become carriers of the infection, harbouring *Streptococcus equi* in chondroids located in the guttural pouch. These carriers are capable of infecting other naïve horses and continue the spread of disease (Chanter *et al.,* 2000; Newton *et al.,* 1997; Newton *et al.,* 2000). Often carriers shed very low numbers of bacteria that are difficult to detect using conventional culture techniques. Therefore, a highly sensitive diagnostic test based on PCR technology would be highly advantageous.

PCR based tests for the detection of *Streptococcus equi* have previously been described, but these have traditionally relied on the detection of the SeM gene (http://www.idexx.com/equine/laboratory/sequi_pcr/sequi_pcrrecommend.jsp) (Sweeney *et al.,* 2005). The SeM gene contains a 5'-region that is unique to *Streptococcus equi.* However, this unique region has been shown to be absent from up to 24% of *Streptococcus equi* isolates recovered from persistently infected horses (Chanter *et al.,* 2000) and to be highly variable in DNA base content (Anzai *et al.,* 2005; Kelly *et al.,* 2006; Waller and Jolley, 2007). This variation may lead to reduced SeM test sensitivity and even the reporting of false negatives, which could have a serious impact on the control of this disease.

ALBER J ET AL: "Multiplex polymerase chain reaction for identification and differentiation of Streptococcus equi subsp zooepidemicus and Streptococcus equi subsp equi" JOURNAL OF VETERINARY MEDICINE SERIES B, vol. 51, no. 10, (2004), pages 455-458, describes methods based on the superoxide dismutase gene and also *see*H and *see*I

BAVERUD V ET AL: "Real-time PCR for detection and differentiation of Streptococcus equi subsp equi and Streptococcus equi subsp zooepidemicus' VETERINARY MICROBIOLOGY, vol. 124, no. 3-4, 2007, pages 219-229, also describes use of the superoxide dismutase *see*I genes.

It will be appreciated that novel diagnostic tests which could mitigate or overcome one or more of these drawbacks would provide a contribution to the art.

### Disclosure of the invention

### Brief description of the invention

At its most general, the present invention provides methods and reagents for detecting the presence or absence of *Streptococcus equi* in a sample, these methods and reagents being based on the assessment of the presence of the *S. equi eqbE* gene sequence in the sample.

Such methods offer the potential for improved sensitivity and specificity compared to existing tests.

The S. *equi eqbE* gene is discussed in a poster entitled "Strangles or Equine Plague? Equibactin, the First Streptococcal Siderophore." (Mitchell et al; American Society of Microbiology's conference on Streptococcal Genetics. St. Malo, France; June 18th-20th 2006). However there is no teaching or suggestion therein of its utility a diagnostic gene for S. *equi.*

A different poster entitled "The evolution of S. equi, results from genome comparisons with S. zooepidemicus" (Mitchell et al; American Society of Microbiology's conference on Streptococcal Genetics. St. Malo, France; June 18th-20th 2006).discusses the comparative genetics of these organisms. However the S. *equi eqbE* gene is not taught or suggested therein for use in the presently claimed invention.

The *S. equi eqbE* gene is also discussed in a poster entitled "A novel streptococcal integrative and conjugative element involved in iron acquisition" (Mitchell et al; XVII Lancefield International Symposium on Streptococci & Streptococcal diseases. Porto Heli, Greece; June 22nd-26th 2008). However there is no teaching or suggestion therein of its utility a diagnostic gene for *S. equi.*

The methods of the invention further include methods of diagnosing or prognosing strangles in a mammal (e.g. canine, or more preferably equine or camelid), which methods comprise assessing the presence of the *S. equi eqbE* gene sequence in a sample from said mammal.

Also provided are the reagents and other materials described herein (e.g. primers and\or probes) for use in such methods, or for use in the preparation of diagnostic or prognostic compositions for such methods.

Determination of whether horses are infected with strangles will be useful in refining management procedures, for example in selecting animals or populations for vaccination, or employing appropriate isolation procedures to limit the risk of such animals spreading infection.

Some particular aspects and embodiments will now be discussed in more detail:

### Sample

The mammal is preferably equine e.g. a horse, donkey or mule. Camelids (or canines) may also be sampled since they may also harbour *S. equi.*

The sample will generally be obtained from an individual animal which is believed to be affected by or a carrier of strangles, or being at risk of these things. For example it may be obtained from symptomatic or asymptomatic, contagious or shedding horses. Nucleic acid containing samples may be obtained from nasal swabs or washes, pus from an abscess and lavages of the guttural pouch, the primary site for asymptomatic carriage (Newton et al, 2000).

The samples may be pooled from herds or other collections.

Different samples may be taken at different time e.g. 0, 7 and 14 days.

The DNA sample analysed may be all or part of the sample being obtained. Methods of the present invention may therefore include obtaining a sample of nucleic acid obtained from the mammal.

Alternatively, the assessment of SEQ ID No 2 may be performed or based on an historical DNA sample, or information already obtained therefrom.

### S. equi eqbE gene sequence

The methods described herein comprise assessing the presence or sequence of all or part of the *S. equi eqbE* gene.

In particular the methods will generally be based on assessing the presence of sequence of an *S. equi eqbE* signature sequence described herein.

The present inventors have defined a 833 bp signature sequence in the *eqbE* gene which is not only apparently unique to *S*. *equi* (and in particular, not present in the closely related *Streptococcus zooepidemicus*) but was also invariant amongst 26 isolates of *S. equi* recovered from horses between 1981 and 2007 and from the USA, Canada, Australia and Europe.

Because this sequence is apparently unique to *Streptococcus equi* and shows no sequence variation across a diverse panel of strains, this 833 bp signature sequence is an ideal candidate upon which to base genetic tests for detecting *Streptococcus equi.*

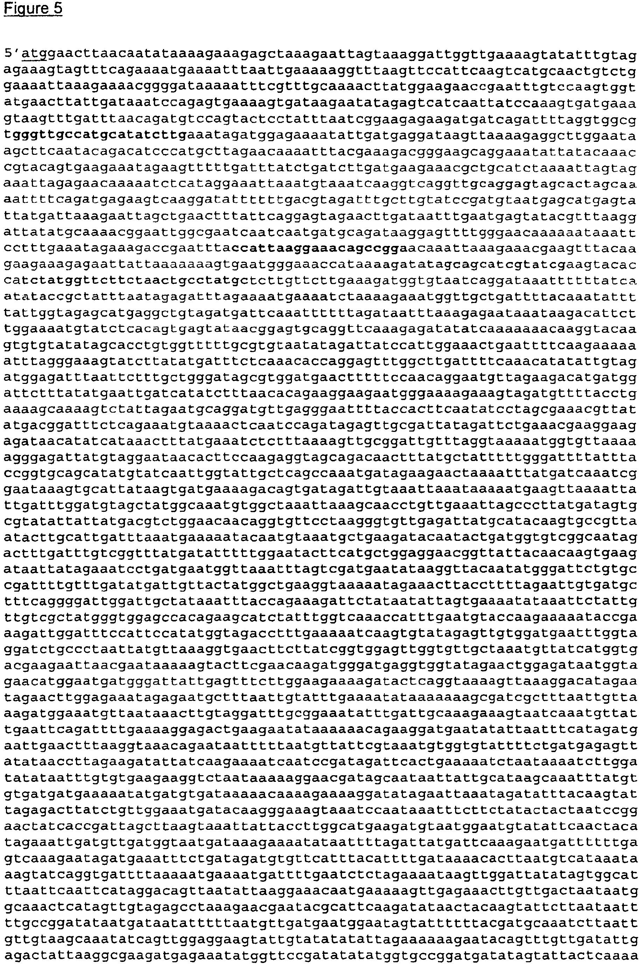

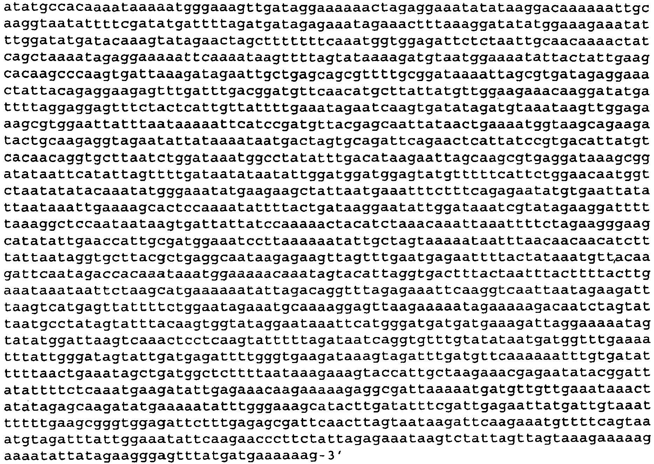

The full CDS of *eqbE* is shown in Figure 5 (SEQ ID No 1).

The non-variable 833 bp S. *equi eqbE* signature sequence is shown within the *eqbE* gene in Figure 5 from positions 276 to 1108 (SEQ ID No 2).

### Preferred methods of the invention

In one aspect a method may comprise:
(i) providing a sample of nucleic acid (e.g. from an equine mammal), and
(ii) establishing the presence or absence of SEQ ID No 2,
(iii) correlating the presence or absence of SEQ ID No 2, with the presence or absence of *S. equi* in the sample.

In one aspect, establishing the presence or absence of SEQ ID No 2 is done by means of a sequence-specific probe. The detection probe will be complementary to a sequence that is present within SEQ ID No 2. Hybridization is carried out under conditions such that the probe binds to SEQ ID No 2 to form a stable hybrid duplex only if the hybridizing regions of the probe is complementary to the nucleic acid in the sample.

In one aspect, establishing the presence or absence of SEQ ID No 2 is done by means of a nucleic acid amplification reaction to amplify all or part of SEQ ID No 2 that may be present in the sample.

The amplification reaction may be performed at the "point-of-care" using methods published in the art. For example US patent application 20090215050 entitled "Systems and methods for point-of-care amplification and detection of polynucleotides" describes the use of solid silicon supports for detecting bacterial infection from blood or nasal swabs. A number of detection methods are described therein including fluorometric, chemiluminescent, and electrochemical. Other systems are described in the literature including e.g. "A novel electrochemical biosensor based on dynamic polymerase-extending hybridization for E. coli O157:H7 DNA detection" Wang et al. (2009) Talanta Volume 78, Issue 3, pages 647-652. This relates to a biosensor having single-stranded DNA (ssDNA) probe functionalized aluminum anodized oxide (AAO) nanopore membranes useful for bacterial pathogen detection.

Preferably the nucleic acid amplification reaction is done by means of two DNA primers to amplify all or part of SEQ ID No 2.

In one aspect of invention relates to a process for detecting SEQ ID No 2 nucleic acid in a sample, wherein the process comprises using PCR to amplify all or part of SEQ ID No 2 that may be present in the sample.

For example the invention provides oligonucleotide primers and probes that enable the amplification of all or part of SEQ ID No 2, and specific detection thereof.

| | | |
|---|---|---|
| eqbE2f: | GGGTTGCCATGCATATCTTG | {Sense} |
| eqbE2r: | TCCGGCTGTTTCCTTAATGG | {Antisense} |

The PCR may be real time PCR where detecting and identifying amplified nucleic acid is achieved by hybridization with one or more sequence-specific oligonucleotide probes. Examples of validated real-time PCR primers and matching probe for the detection of this non-variable region of the *eqbE* gene of *Streptococcus equi* are provided herein.

| | | |
|---|---|---|
| EqbEf: | AAGATATAGCAGCATCGTATCG | {Sense} |
| EqbEr: | TCTAAATCTCTATTAAATAGCGGTATATTG | {Antisense} |

Equidetectin probe: 5' (6-Fam) TCT+ATG+GTT+CTT+CTAACTGCCTATGC (BHQ1)

The use of such a real time PCT system is preferred since it provides high specificity (the primers and probe only generate a detectable PCR product when DNA from *Streptococcus equi* was used - see Figure 1) and high sensitivity (the preferred primers and probe of this invention could detect as little as 10 copies of *Streptococcus equi* DNA by real-time PCR assay and compared well with existing methods of diagnosing *S. equi* infection - see Figure 3 and Figure 4).

Some of these methods will now be described in more detail.

In all cases the herein, one or more of the probes or primers may be labelled.

Where the term "label" or "labelled" is used herein this refers to a detectable molecule which is incorporated indirectly or directly into an oligonucleotide, wherein the label molecule facilitates the detection of the oligonucleotide. Methods of producing labelled probes or primers are well known to those skilled on the art (See for example, Molecular Cloning, a laboratory manual: editors Sambrook, Fritsch, Maniatis; Cold Spring Harbor Laboratory Press, 1989; BioTechniques "Producing single-stranded DNA probes with the Taq DNA polymerase: a high yield protocol," 10:36, 1991). Alternatively, the detectable moiety may be incorporated directly or indirectly such as, for example, by biotinylating the 5' aminogroup of the oligonucleotide with sulfo-NHS-biotin. Other label molecules, known to those skilled in the art as being useful for detection, include radioactively, fluorescently, enzymatically or electrochemically labelled molecules.

Various fluorescent molecules are known in the art which are suitable for use to label a nucleic acid substrate for the method of the present invention. Fluorescent molecules used as labels may include amine-reactive molecules which are reactive to end terminal amines of the substrate; sulfonyl chlorides which are conjugated to the substrate through amine residues; and the like. Depending on the fluorescent molecule used, incorporating the substrate with the fluorescent molecule label include attachment by covalent or noncovalent means. The protocol for such incorporation may vary depending upon the fluorescent molecule used. Such protocols are known in the art for the respective fluorescent molecule.

A preferred label is Fam.

### Probing

The method of assessment of the SEQ ID No 2 may comprise directly determining the binding of an oligonucleotide probe to the nucleic acid sample. The probe may comprise a nucleic acid sequence which hybridizes specifically to a distinctive part of SEQ ID No 2.

The term "hybridization" refers to the formation of a duplex structure by two single-stranded nucleic acids due to complementary base pairing. Hybridization can occur between complementary nucleic acid strands or between nucleic acid strands that contain minor regions of mismatch. Conditions under which only fully complementary nucleic acid strands will hybridize are referred to as "stringent hybridization conditions". Two single-stranded nucleic acids that are complementary except for minor regions of mismatch are referred to as "substantially complementary". Stable duplexes of substantially complementary sequences can be achieved under less stringent hybridization conditions. Those skilled in the art of nucleic acid technology can determine duplex stability empirically considering a number of variables including, for example, the length and composition of the oligonucleotides, ionic strength, and incidence and type of mismatched base pairs.

Where the nucleic acid is double-stranded DNA, hybridisation will generally be preceded by denaturation to produce single-stranded DNA. A screening procedure, chosen from the many available to those skilled in the art, is used to identify successful hybridisation events and isolated hybridised nucleic acid.

Probing may employ the standard Southern blotting technique. For instance DNA may be extracted from cells and digested with different restriction enzymes. Restriction fragments may then be separated by electrophoresis on an agarose gel, before denaturation and transfer to a nitrocellulose filter. Labelled probe may be hybridised to the DNA fragments on the filter and binding determined.

Binding of a probe to target nucleic acid (e.g. DNA) may be measured using any of a variety of techniques at the disposal of those skilled in the art. For instance, probes may be radioactively, fluorescently, enzymatically or electrochemically labelled as described above.

The term "probe" refers to an oligonucleotide which forms a duplex structure with a sequence of a target nucleic acid due to complementary base pairing. The probe will consist of a "hybridizing region", which is a region of the oligonucleotide preferably consisting of 10 to 50 nucleotides, more preferably from 15 to 30 nucleotides, corresponding to a region of the target sequence. "Corresponding" means identical to or complementary to the designated nucleic acid. An oligonucleotide probe optionally can be bound to additional molecules which allow for the detection or immobilization of the probe but do not alter the hybridization characteristics of the probe. One of skill in the art will recognize that, in general, the complement of an oligonucleotide probe is also suitable as a probe.

Preferably, the lengths of these probes are at least 15 to 30 nucleotides. After incubation, all non-annealed nucleic acids are removed from the nucleic acid:gene hybrid. The presence of nucleic acids that have hybridized, if any such molecules exist, is then detected. Using such a detection scheme, the nucleic acid from the cell type or tissue of interest can be immobilized, for example, to a solid support such as a membrane, or a plastic surface such as that on a microtitre plate or polystyrene beads. In this case, after incubation, non-annealed, labeled nucleic acid reagents are easily removed. Detection of the remaining, annealed, labeled nucleic acid reagents is accomplished using standard techniques well-known to those in the art. The gene sequences to which the nucleic acid reagents have annealed can be compared to the annealing pattern expected from a normal gene sequence in order to determine whether a gene mutation is present.

As discussed above, suitable probes may comprise all or part of the SEQ ID No 2 sequence (or reverse complement thereof).

Those skilled in the art are well able to employ suitable conditions of the desired stringency for selective hybridisation, taking into account factors such as oligonucleotide length and base composition, temperature and so on.

Suitable selective hybridisation conditions for oligonucleotides of 17 to 30 bases include hybridization overnight at 42°C in 6X SSC and washing in 6X SSC at a series of increasing temperatures from 42°C to 65°C. One common formula for calculating the stringency conditions required to achieve hybridization between nucleic acid molecules of a specified sequence homology is (Sambrook et al., 1989): Tₘ = 81.5°C + 16.6Log [Na+] + 0.41 (% G+C) - 0.63 (% formamide) - 600/#bp in duplex.

Other suitable conditions and protocols are described in Molecular Cloning: a Laboratory Manual: 2nd edition, Sambrook et al., 1989, Cold Spring Harbor Laboratory Press and Current Protocols in Molecular Biology, Ausubel et al. eds., John Wiley & Sons, 1992.

### Amplification-based methods

Preferred detection methods of the invention are based on PCR or other amplification procedures wherein, if present, all or part of SEQ ID No 2 is amplified.

The existence (and preferably identity) of any amplification product may then be assessed by any suitable method, e.g., as described herein. An example of such a method is a combination of PCR and low stringency hybridisation with a suitable probe. Unless stated otherwise, the methods of assessing the presence of SEQ ID No 2 described herein may be performed on a native DNA sample, or on an amplification product thereof.

Where the method involves PCR, or other amplification procedure, any suitable SEQ ID No 2-amplifying primers may be used. Preferably the primers both bind within SEQ ID No 2, though one or both may flank SEQ ID No 2, provided some or all of SEQ ID No 2 is amplified.

The term "primer" refers to an oligonucleotide, whether natural or synthetic, capable of acting as a point of initiation of DNA synthesis under conditions in which synthesis of a primer extension product complementary to a nucleic acid strand is induced, i.e., in the presence of four different nucleoside triphosphates and an agent for polymerization (i.e., DNA polymerase or reverse transcriptase) in an appropriate buffer and at a suitable temperature. A primer need not reflect the exact sequence of the template but must be sufficiently complementary to hybridize with a template. Primers can incorporate additional features which allow for the detection or immobilization of the primer but do not alter the basic property of the primer, that of acting as a point of initiation of DNA synthesis.

An oligonucleotide primer for use in nucleic acid amplification may be about 30 or fewer nucleotides. Generally specific primers are upwards of 14 nucleotides in length, but are preferably 15-35 inclusive, more preferably 18-32, more preferably 20-30. Those skilled in the art are well versed in the design of primers for use processes such as PCR. Various techniques for synthesizing oligonucleotide primers are well known in the art, including phosphotriester and phosphodiester synthesis methods.

Preferably the amplified region (including some of SEQ ID No 2) which the primers flank is less than 600, 500, 400, 300 nucleotides, more preferably less than 250 nucleotides, more preferably 20 to 200, or 50 to 180, or 100 to 150 nucleotides in length.

Suitable polymerase chain reaction (PCR) methods are reviewed, for instance, in "PCR protocols; A Guide to Methods and Applications", Eds. Innis et al, 1990, Academic Press, New York, Mullis et al, Cold Spring Harbor Symp. Quant. Biol., 51:263, (1987), Ehrlich (ed), PCR technology, Stockton Press, NY, 1989, and Ehrlich et al, Science, 252:1643-1650, (1991)). PCR comprises steps of denaturation of template nucleic acid (if double-stranded), annealing of primer to target, and polymerisation.

An amplification method may be a method other than PCR. Such methods include strand displacement activation, the QB replicase system, the repair chain reaction, the ligase chain reaction, rolling circle amplification and ligation activated transcription. For convenience, and because it is generally preferred, the term PCR is used herein in contexts where other nucleic acid amplification techniques may be applied by those skilled in the art. Unless the context requires otherwise, reference to PCR should be taken to cover use of any suitable nucleic amplification reaction available in the art. As noted above, this includes (without limitation) so called "point of care" amplification reactions.

Examples of results from the real time PCR genotyping assay are shown below.

### Sequencing

The presence of SEQ ID No 2 may be assessed or confirmed by nucleotide sequencing of a nucleic acid sample to determine whether all that sequence, or a characteristic portion, is present.

Nucleotide sequence analysis may be performed on a genomic DNA sample, or amplified part thereof, or RNA sample as appropriate, using methods which are standard in the art. Example sequence primers are described herein.

Other techniques which may be used are single base extension techniques and pyrosequencing.

### Primers and probes

Probes and primers for use in the methods form aspects of the present invention form a further aspect of the invention.

For example in one aspect there is provided a pair of nucleic acid primers which primers are adapted to amplify 833, or more than 800, 700, 600, 500, 400, 300, 200, 150, 100, 90, 80, 70, 60, 50, 40, 30, or 20 contiguous nucleotides of SEQ ID No 2.

As noted above, the primers may themselves bind specifically to SEQ ID No 2, or one or both may flank that sequence. If flanking primers are used, then some of or all of the *eqbE* gene outside of SEQ ID No 2 will also be amplified.

Preferably the amplified product, including primers and any sequence outside of SEQ ID No 2 is less than 850, 800, 700, 600, 500, 400, 300, 200, 150, 100, 90, 80, 70, 60 bp in length.

Preferred primers include eqbE2f; eqbE2r (pair) and EqbEf; EqbEr (pair) plus complements and reverse complements thereof. As is understood by those skilled in the art, a 'complement' or 'complementary' or 'reverse complement' sequence (the terms are equivalent) is one which is the same length as a reference sequence, but is 100% complementary thereto whereby by each nucleotide is base paired to its counterpart running in anti-parallel fashion i.e. G to C, and A to T or U.

Preferred probes include the Equidetectin probe

### Kits

Nucleic acid for use in the methods of the present invention, such as an oligonucleotide probe and/or pair of amplification primers useful for the amplification of all or part of SEQ ID No 2, and specific detection thereof, may be provided in isolated form and may be part of a kit, e.g. in a suitable container such as a vial in which the contents are protected from the external environment. The kit may include instructions for use of the nucleic acid, e.g. in PCR and/or a method for determining the presence of nucleic acid of interest in a test sample and/or in the detection of *S. equi.* Primers "substantially complementary" to these are also included. As known to those skilled in the art, a very high degree of complementarity is needed for specificity and sensitivity involving hybridization, although it need not be 100%. Thus, for example, an oligonucleotide which is identical in nucleotide sequence to an oligonucleotide disclosed herein, except for one base change or substitution, may function equivalently to the disclosed oligonucleotides.

A kit wherein the nucleic acid is intended for use in PCR may include one or more other reagents required for the reaction, such as polymerase, nucleotides, buffer solution etc. A kit for use in determining the presence or absence of nucleic acid of interest may include one or more articles and/or reagents for performance of the method, such as means for providing the test sample itself, e.g. a nasal swab (such components generally being sterile).

### Combination tests

The method of the invention may optionally comprise, in addition to assessing SEQ ID No 2, the assessment from the same sample of other diagnostic or prognostic markers which are linked or associated with other equine disorders or pathogens.

Particular methods of detecting SEQ ID No 2 in nucleic acid samples are described in more detail hereinafter.

Any sub-titles herein are included for convenience only, and are not to be construed as limiting the disclosure in any way.

The invention will now be further described with reference to the following non-limiting Figures and Examples. Other embodiments of the invention will occur to those skilled in the art in the light of these.

The disclosure of all references cited herein, inasmuch as it may be used by those skilled in the art to carry out the invention, is hereby specifically incorporated herein by cross-reference.

### Figures

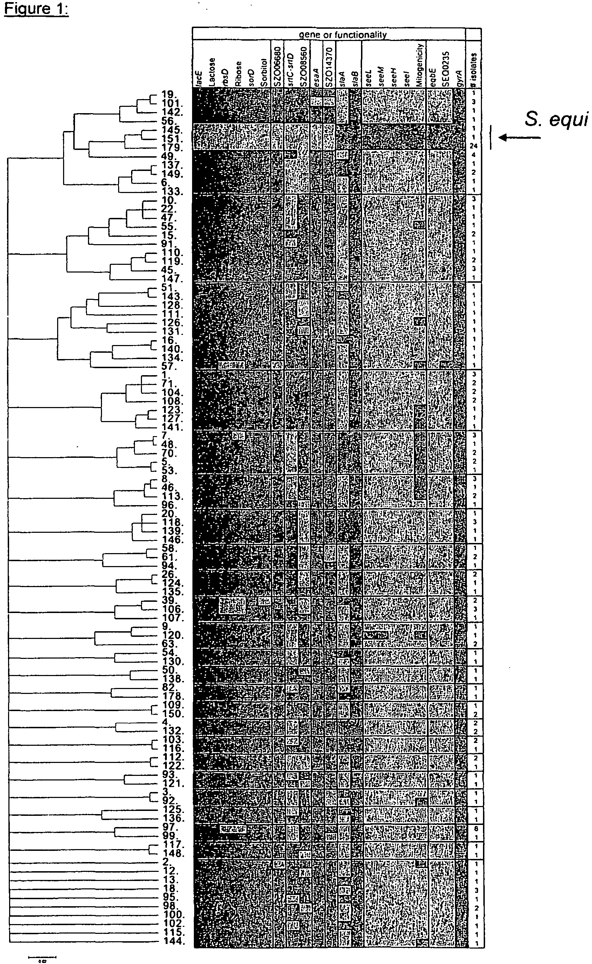

Figure 1: ClonalFrame phylogenetic tree of 26 *S*. *equi* and 142 *S*. *zooepidemicus* isolates and its relationship with the prevalence of selected differences between the *Streptococcus equi* 4047 and *Streptococcus zooepidemicus* H70 genomes. Genes shown are *lacE, rbsD, sorD,* SZO06680 (encoding a putative hyaluronate lyase and specific to the 4 bp missing from SEQ1479), *srtC, srtD,* SZO08560 (encoding an lnlA-like domain), SZO14370 (within the CRISPR locus), *slaA, slaB, seeL, seeM, seeH*, *seeI*, *eqbE,* SEQ0235 (encoding Se18.9) and *gyrA.* Functional assays determined the ability of different isolates to ferment lactose, ribose and sorbitol and to induce mitogenic responses in equine PBMCs. The number of isolates representing each multilocus sequence type (ST) is indicated. STs where all isolates contained the gene or possessed functional activity, STs where all isolates lacked the gene or functionality, and STs containing some isolates containing the gene or functionality and some that did not are shaded.

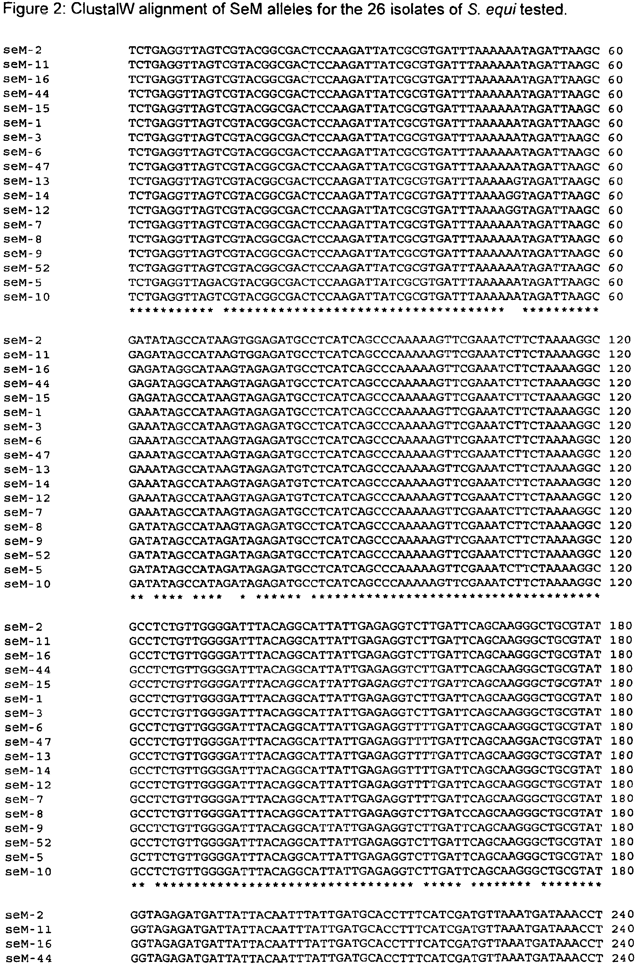

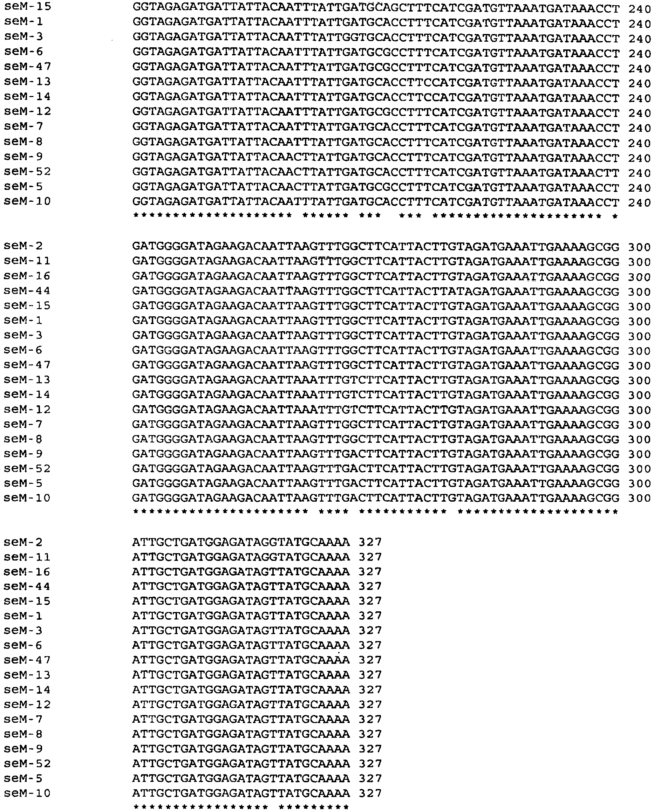

Figure 2: ClustalW alignment of SeM alleles for the 26 isolates of S. *equi* tested.

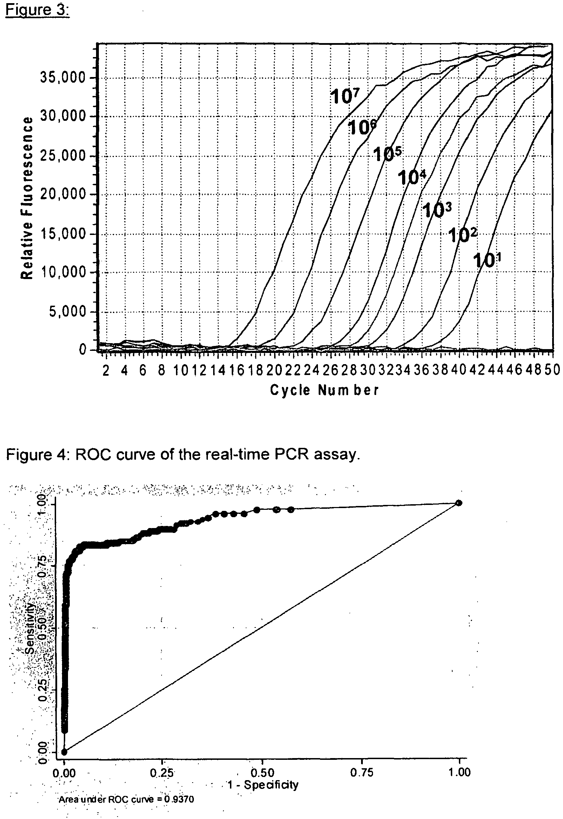

Figure 3: Standard curve for real-time PCR assay using *eqbE* primers and probe. The real-time PCR curve generated from DNA prepared from a clinical sample is shown.
Figure 4: ROC curve of the real-time PCR assay.
Figure 5: The full CDS of *eqbE* is shown with the non-variable region highlighted with the atg translational start underlined. Primers zm435 zm436 and zm437 used to sequence this region of *eqbE* are shown. Diagnostic PCR primers eqbE2f and eqbE2r are highlighted. Real time PCR primers EqbEf and EqbEr are shown and the equidetectin probe is shown.

### Sequences in listing

- 1: EqbE - complete CDS
- 2: EqbE - non variable signature sequence
- 3: EqbE - amplified product
- 4: EqbE - amplified product#2
- 5: Diagnostic primer - F
- 6: Diagnostic primer - R
- 7: Real time primer - F
- 8: Real time primer - R
- 9: Real time probe

### Examples

### Example 1 - identification of genes specific to Streptococcus equi

The inventors compared the genome sequences of *Streptococcus equi* strain 4047 and *Streptococcus zooepidemicus* strain H70 and identified 60 alternative loci containing genes that are unique to *Streptococcus equi.*

Following the initial comparison of these two strains, the inventors determined the prevalence of these loci across a diverse panel of 26 isolates of *Streptococcus equi* and 142 isolates of *Streptococcus zooepidemicus* (Figure 1). The 26 *S. equi* strains were isolated from strangles cases between 1981 and 2008 across several continents and represented 3 different MLST sequence types (Webb *et al.,* 2008) and 18 different SeM alleles (Kelly *et al.,* 2006) (Figure 2).

Through this analysis the inventors then identified a 63 kb locus (lCESe2) containing a 14 gene region present in all strains of *Streptococcus equi* that was absent from all diverse strains of *Streptococcus zooepidemicus* and encoded a putative non-ribosomal peptide synthesis (NRPS) system. The *Streptococcus equi* locus has most overall similarity to the NRPS cluster 1 of *Clostridium kluyveri,* which is proposed to biosynthesise a putative siderophore (Seedorf *et al.,* 2008). Several of the encoded proteins were also similar to the NRPS complex of *Yersinia sp.* that produces the ferric iron-binding siderophore yersiniabactin (Gehring et al., 1988).

The inventors considered that the locus represented a potentially advantageous choice for a diagnostic target because it is likely to produce an intracellular enzyme that is less likely to be targeted by the equine immune response and in turn is less likely to be of variable sequence between different strains of *Streptococcus equi.*

The inventors sequenced internal fragments of *eqbE* and identified a region of 833 bp in which there was no sequence variation among 26 isolates of *Streptococcus equi* recovered from horses between 1981 and 2007 and from the USA, Canada, Australia and Europe, suggesting that this part of the *eqbE* gene is an ideal candidate for the development of a new PCR diagnostic test for *Streptococcus equi.*

### Example 2 - validation data for the real time PCR assay for the detection of Streptococcus equi.

The objectives of this Example were:
- Compare real-time PCR test results with PCR combined with culture, which is considered the gold standard method.
- Calculate the cut-off point of the real-time PCR to consider the test positive and the sensitivity and specificity associated to that cut-off.

### Methods

The presence of the *eqbE* non-variable region was determined in clinical samples by real-time PCR using a 6-Fam-labelled probe (equidetectin) and the primers EqbEf and EqbEr on a Techne Quantica instrument. For the PCR, 2 µl DNA extracted from clinical samples was mixed with 0.6 µl of 10 µM EqbEf and EqbEr primers (Sigma), 10 µl QPCR ROX mix (Abgene), 1.5 µl of 2 µM equidetectin (Sigma) and 5.3 µl of water to give a total volume of 20 µl and subjected to thermocycling at 105°C for 5 min, 95°C for 15 minutes followed by 50 cycles of 95°C for 15 seconds and 60°C for 30 seconds. Data were analysed using Quansoft software (Techne). Crossing point values relative to known standards were used to calculate the number of copies of *eqbE* in the clinical sample. Figure 3 shows an example of a typical positive clinical sample, which contains between 1000 and 10,000 copies of the *eqbE* gene.

### Data and analysis

Data comprised information on a total of diagnostic samples that had been processed by real-time PCR with (n=1057). Of the 1057 samples, 1055 had been previously PCR tested using the conventional current diagnostic PCR and 983 had also had culture conducted on the samples. For the purposes of these analyses a dichotomous gold standard diagnostic variable (goldstandard) was created which corresponded to a value of 0 for culture and diagnostic PCR negative and 1 for culture and/or diagnostic PCR positive. Data for real-time PCR came as duplicate and the average of both readings were calculated. Data for real-time PCR also came in 2 forms. The first form were continuous variables (copyav) representing the number of DNA copies quantified by real-time PCR and the second were ordered categorical variables (qpcrcat) that was categorised as 0 for negative by real-time PCR, 1 for 20-100 copies and 2 for >100 copies. Re-classification was performed of the categorical variables into binary variables around the arbitrary cut-off of 100 copies (qpcrbin) such that 0 represented <100 copies and 1 represented >100 copies.

Data were supplied in the form of an Excel spreadsheet (Spreadsheet for PCR_qPCR_culture for strangles.xls), which following some minor amendments were transferred to a Stata 8.0 data file for analysis.

Cross tabulations were performed using qpcrbin as new assay variables and goldstandard as the gold standard assay. From these we can use the % figures in the second and third rows of each cell as various test characteristic measures. These are explained as follows:
i) Sensitivity = % of true positives that test positive (100-sensitivity = % false negative)
ii) Specificity = % of true negatives that test negative (100-specificity = % false positive)
iii) NPV = predictive value of a negative test = % of test negatives that are truly negative
iv) PPV = predictive value of a positive test = % of test positives that are truly positive

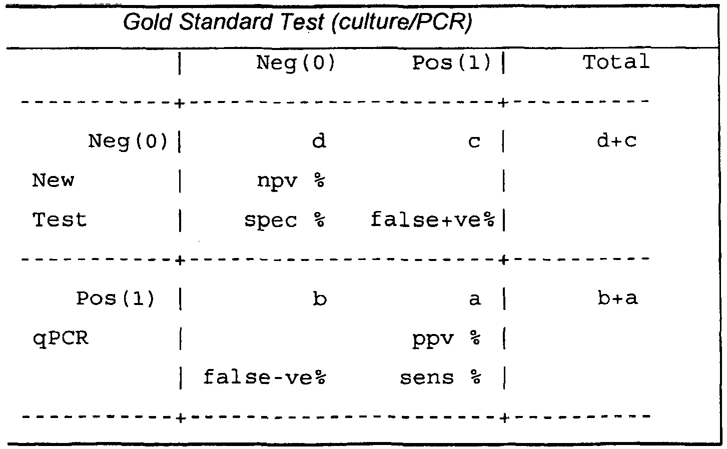

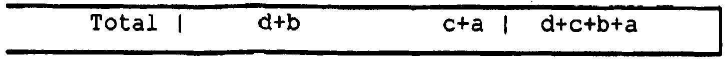

Data were also analysed using Receiver Operating Characteristics (usually shortened to ROC) commands in Stata. This analysis method generates summary data (including graph and tables presented below) for sensitivity and specificity estimates for all the various cutoff points within the data based in this instance on the copy number (copyave) data applied against the gold standard.

### Results

Figure 4 shows the ROC curve that compares the real-time PCR with the gold standard test. It quantifies the accuracy of the new test, as the higher area under the curve the better performance of the test:
- 0.90-1 = excellent
- 0.80-0.90 = good
- 0.70-0.80 = fair
- 0.60-0.70 = poor
- 0.50-0.60 = fail

In this case the area under the curve is 0.94 that represents an excellent accuracy of the real-time PCR because the area measures the ability of the test to correctly classify those with and without positive results from other tests.

Table 1 summarises from the detailed data outputs presented below the sensitivity and specificity at a series of copy number thresholds for the real-time PCR data. The cut-off point of the diagnosis test should be the one with highest sensitivity and specificity. The sensitivity of a test is the proportion of animals with the disease that have a positive test result and the specificity of the test is the proportion of animals without the disease that have a negative test. Therefore we are interested in having the highest sensitivity possible.

**Table 1: Summary of sensitivity and specificity estimates for various S. equi real-time PCR copy thresholds**

| | | |
|---|---|---|
| | | |

| **Copy threshold (≥)** | **Sensitivity (%)** | **Specificity (%)** |
|---|---|---|
| 1 | 97.6 | 42.6 |
| 20 | 89.8 | 74.6 |
| 50 | 84.3 | 87.2 |
| 98 | 83.5 | 93.6 |
| 150 | 82.7 | 95 |
| 200 | 88 | 93 |

Table 2 represents the cross tabulation between qpcrbin and the goldstandard. The Sensitivity of the test is 83.5% and the specificity is 93.6%. The percentage of test negatives that are truly negative is 97.6% and the predictive value of a positive test is 63.8%.

**Table 2: Sensitivity and Specificity of the real-time PCR in Binary form for a cut-off point of 100 copies**

| *goldstandard* | | | |
|---|---|---|---|
| qpcrbin | 0 | 1 | Total |
| | | | |
| 0 | 870 | 21 | 891 |
| | 97.64 | 2.36 | 100.00 |
| | 93.55 | 16.54 | 84.30 |
| | | | |
| 1 | 60 | 106 | 166 |
| | 36.14 | 63.86 | 100.00 |
| | 6.45 | 83.46 | 15.70 |
| | | | |
| Total | 930 | 127 | 1,057 |
| | 87.98 | 12.02 | 100.00 |
| | 100.00 | 100.00 | 100.00 |

Based on these data the optimal copy threshold value appears to lie somewhere between 50 and 200 with a threshold of i) 100 copies providing a sensitivity of 83.5% and specificity of 93.6% and ii) 150 copies giving both a sensitivity of 83% and specificity of 95%.

### References

Anzai, T., Kuwamoto, Y., Wada, R., Sugita, S., Kakuda, T., Takai, S., Higuchi, T., and Timoney, J.F. (2005) Variation in the N-terminal region of an M-like protein of Streptococcus equi and evaluation of its potential as a tool in epidemiologic studies. Am J Vet Res 66: 2167-2171.
Chanter, N., Talbot, N.C., Newton, J.R., Hewson, D., and Verheyen, K. (2000) Streptococcus equi with truncated M-proteins isolated from outwardly healthy horses. Microbiology 146 (Pt 6): 1361-1369.
Kelly, C., Bugg, M., Robinson, C., Mitchell, Z., Davis-Poynter, N., Newton, J.R., Jolley, K.A., Maiden, M.C., and Waller, A.S. (2006) Sequence variation of the SeM gene of Streptococcus equi allows discrimination of the source of strangles outbreaks. J Clin Microbiol 44: 480-486.
Newton, J.R., Wood, J.L., Dunn, K.A., DeBrauwere, M.N., and Chanter, N. (1997) Naturally occurring persistent and asymptomatic infection of the guttural pouches of horses with Streptococcus equi. Vet Rec 140: 84-90.
Newton, J.R., Verheyen, K., Talbot, N.C., Timoney, J.F., Wood, J.L., Lakhani, K.H., and Chanter, N. (2000) Control of strangles outbreaks by isolation of guttural pouch carriers identified using PCR and culture of Streptococcus equi. Equine Vet J 32: 515-526.
Seedorf, H., Fricke, W.F., Veith, B., Bruggemann, H., Liesegang, H., Strittmatter, A., Miethke, M., Buckel, W., Hinderberger, J., Li, F., Hagemeier, C., Thauer, R.K., and Gottschalk, G. (2008) The genome of Clostridium kluyveri, a strict anaerobe with unique metabolic features. Proc Natl Acad Sci U S A 105: 2128-2133.
Sweeney, C.R., Timoney, J.F., Newton, J.R., and Hines, M.T. (2005) Streptococcus equi infections in horses: guidelines for treatment, control, and prevention of strangles. J Vet Intern Med 19: 123-134.
Timoney, J.F. (1993) Strangles. Vet Clin North Am Equine Pract 9: 365-374.
Waller, A.S., and Jolley, K.A. (2007) Getting a grip on strangles: recent progress towards improved diagnostics and vaccines. Vet J 173: 492-501.
Webb, K., Jolley, K.A., Mitchell, Z., Robinson, C., Newton, J.R., Maiden, M.C., Waller, A. (2008) Development of an unambiguous and discriminatory multilocus sequence typing scheme for the Streptococcus zooepidemicus group. Microbiology 154:3016-24.

### SEQUENCE LISTING

<120> Diagnostic Test for Streptococcus equi
<130> smk/lp6657092
<160> 9
<170> PatentIn version 3.5
<210> 1
   <211> 6081
   <212> DNA
   <213> Streptococcus equi
<220>
   <221> misc_feature
   <222> (276)..(1108)
<400> 1
   atggaactta acaatataaa agaaagagct aaagaattag taaaggattg gttgaaaagt        60
   atatttgtag agaaagtagt ttcagaaaat gaaaatttaa ttgaaaaagg tttaagttcc       120
   attcaagtca tgcaactgtc tggaaaatta aagaaaacgg ggataaaaat ttcgtttgca       180
   aaacttatgg aagaaccgaa tttgtccaag tggtatgaac ttattgataa atccagagtg       240
   aaaagtgata agaatataga gtcatcaatt atccaaagtg atgaaagtaa gtttgattta       300
   acagatgtcc agtactccta tttaatcgga agagaagatg atcagatttt aggtggcgtg       360
   ggttgccatg catatcttga aatagatgga gaaaatattg atgaggataa gttaaaagag       420
   gcttggaata agcttcaata cagacatccc atgcttagaa caaaatttac gaaagacggg       480
   aagcaggaaa tattatacaa accgtacagt gaagaaatag aagtttttga tttatctgat       540
   cttgatgaag aaacgctgca tctaaaatta gtagaaatta gagaacaaaa atctcatagg       600
   aaattaaatg taaatcaagg tcaggttgca ggagtagcac tagcaaaatt ttcagatgag       660
   aagtcaagga tattttttga cgtagatttg cttgtatccg atgtaatgag catgagtatt       720
   atgattaaag aattagctga actttattca ggagtagaac ttgataattt gaatgagtat 780
   acgtttaagg attatatgca aaacggaatt ggcgaatcaa tcaatgatgc agataaggag 840
   ttttgggaac aaaaaataaa ttcctttgaa atagaaagac cgaatttacc attaaggaaa 900
   cagccggaac aaattaaaga aacgaagttt acaagaagaa agagaattat taaaaaaagt 960
   gaatgggaaa ccataaaaga tatagcagca tcgtatcgaa gtacaccatc tatggttctt 1020
   ctaactgcct atgctcttgt tcttgaaaga tggtgtaatc aggataaatt ttttatcaat 1080
   ataccgctat ttaatagaga tttagaaaat gaaaatctaa aagaaatggt tgctgatttt 1140
   acaaatattt tattggtaga gcatgaggct gtagatgatt caaatttttt agataattta 1200
   aagagaataa ataagacatt cttggaaaat gtatctcaca gtgagtataa cggagtgcag 1260
   gttcaaagag atatatcaaa aaaacaaggt acaagtgtgt atatagcacc tgtggttttt 1320
   gcgtgtaata tagattatcc attggaaact gaattttcaa gaaaaaattt agggaaagta 1380
   tcttatatga tttctcaaac accaggagtt tggcttgatt ttcaaacata tattgtagat 1440
   ggagatttaa ttctttgctg ggatagcgtg gatgaacttt ttccaacagg aatgttagaa 1500
   gacatgatgg attctttata tgaattgatc atatctttaa cacagaagga agaatgggaa 1560
   aagaaagtag atgttttacc tgaaaagcaa aagtctatta gaatgcagga tgttgaggga 1620
   attttaccac ttcaatatcc tagcgaaacg ttatatgacg gatttctcag aaatgtaaaa 1680
   ctcaatccag atagagttgc gattatagat tctgaaacga aggaagagat aacatatcat 1740
   aaactttatg aaatctcttt aaaagttgcg gattgtttag gtaaaaatgg tgttaaaaag 1800
   ggagattatg taggaataac acttccaaga ggtagcagac aactttatgc tatttttggg 1860
   attttattta ccggtgcagc atatgtatca attggtattg ctcagccaaa tgatagaaga 1920
   actaaaattt atgatcaaat cggaataaag tgcattataa gtgatgaaaa gacagtgata 1980
   gattgtaaat taaataaaaa tgaagttaaa attattgatt tggatgtagc tatggcaaat 2040
   gtggctaaat taaagcaacc tgttgaaatt agcccttatg atagtgcgta tattattatg 2100
   acgtctggaa caacaggtgt tcctaagggt gttgagatta tgcatacaag tgccgttaat 2160
   acttgcattg atttaaatga aaaatacaat gtaaatgctg aagatacaat actgatggtg 2220
   tcggcaatag actttgattt gtcggtttat gatatttttg gaatacttca tgctggagga 2280
   acggttatta caacaagtga agataattat agaaatcctg atgaatggtt aaatttagtc 2340
   gatgaatata aggttacaat atgggattct gtgccgattt tgtttgatat gattgttact 2400
   atggctgaag gtaaaaatag aaacttacct tttagaattg tgatgctttc aggggattgg 2460
   attgctataa atttaccaga aagattctat aatattagtg aaaatataaa ttctattgtt 2520
   gtcgctatgg gtggagccac agaagcatct atttggtcaa accatttgaa tgtaccaaga 2580
   aaaataccga aagattggat ttccattcca tatggtagac ctttgaaaaa tcaagtgtat 2640
   agagttgtgg atgaatttgg taggatctgc cctaattatg ttaaaggtga acttcttatc 2700
   ggtggagttg gtgttgctaa atgttatcat ggtgacgaag aattaacgaa taaaaagtac 2760
   ttcgaacaag atgggatgag gtggtataga actggagata atggtagaac atggaatgat 2820
   gggattattg agtttcttgg aagaaaagat actcaggtaa aagttaaagg acatagaata 2880
   gaacttggag aaatagagaa tgctttaatt gtatttgaaa atataaaaaa agcgatcgct 2940
   ttaattgtta aagatggaaa tgttaataaa cttgtaggat ttgcggaaat atttgattgc 3000
   aaagaaagta atcaaatgtt attgaattca gattttgaaa aggagactga agaatataaa 3060
   aaacagaagg atgaatatat taatttcata gatgaattga actttaaggt aaacagaata 3120
   atttttaatg ttattcgtaa atgtggtgta ttttctgatg agagttatat aaccttagaa 3180
   gatattatca agaaaatcaa tccgatagat tcactgaaaa atctaataaa atcttggata 3240
   tataatttgt gtgaagaagg tctaataaaa aggaacgata gcaataatta ttgcataagc 3300
   aaatttatgt gtgatgatga aaaatatgat gtgataaaaa caaaagaaaa ggatatagaa 3360
   ttaaatagat atttacaagt attagagact tatctgttgg aaatgataca agggaaagta 3420
   aatccaataa atttcttcta tactactaat ccggaactat caccgattag cttaagtaaa 3480
   ttattacctt ggcatgaaga tgtaatggaa tgtatattca actacataga aattgatgtt 3540
   gatggtaatg ataaagaaaa tataatttta gattatgatt caaagaatga ttttttgagt 3600
   caaagaatag atgaaatttc tgatagatgt gttcatttac attttgataa aacacttaat 3660
   gtcataaata aagtatcagg tgattttaaa aatgaaaatg attttgaatc tctagaaaat 3720
   aagttggatt atatagtggc atttaattca attcatagga cagttaatat taaggaaaca 3780
   atgaaaaagt tgagaaactt gttgactaat aatggcaaac tcatagttgt agagcctaaa 3840
   gaacgaatac gcattcaaga tataactaca agtattctta ataattttgc cggatataat 3900
   gataatattt ttaatgttga tgaatggaat agtatttttt acgatgcaaa tcttaattgt 3960
   tgtaagcaaa tatcagttgg aggaagtatt gtatatatat tagaaaaaag aatacagttt 4020
   gttgatattg agactattaa ggcgaagatg agaaatatgg ttccgatata tatggtgccg 4080
   gatgatatag tattactcaa aaatatgcca caaaataaaa atgggaaagt tgataggaaa 4140
   aaactagagg aaatatataa ggacaaaaaa ttgcaaggta atattttcga tatgatttta 4200
   gatgatagag aaatagaaac tttaaaggat atatggaaag aaatatttgg atatgataca 4260
   aagtatagaa ctagcttttt ttcaaatggt ggagattctc taattgcaac aaaactatca 4320
   gctaaaatag aggaaaaatt caaaataagt tttagtataa aagatgtaat ggaaaatatt 4380
   actattgaag cacaagccca agtgattaaa gatagaattg ctgagcagcg ttttgcggat 4440
   aaaattagcg tgatagagga aactattaca gaggaagagt ttgatttgac ggatgttcaa 4500
   catgcttatt atgttggaag aaacaaggat atgattttag gaggagtttc tactcattgt 4560
   tattttgaaa tagaatcaag tgatatagat gtaaataagt tggagaaagc gtggaattat 4620
   ttaataaaaa ttcatccgat gttacgagca attataactg aaaatggtaa gcagaagata 4680
   ctgcaagagg tagaatatta taaaataatg actagtgcag attcagaact cattatccgt 4740
   gacattatgt cacaacaggt gcttaatctg gataaatggc ctatatttga cataagaatt 4800
   agcaagcgtg aggataaagc ggatataatt catattagtt ttgataatat aatattggat 4860
   ggatggagta tgtttttcat tctggaacaa tggtctaata tatacaaata tgggaaatat 4920
   gaagaagcta ttaatgaaat ttctttcaga gaatatgtga attatattaa taaattgaaa 4980
   agcactccaa aatattttac tgataaggaa tattggataa atcgtataga aggattttta 5040
   aaggctccaa taataagtga ttattatcca aaaactacat ctaaacaaat taaattttct 5100
   agaagggaag catatattga accattgcga tggaaatcct taaaaaatat tgctagtaaa 5160
   aataatttaa caacaacatc tttattaata ggtgcttacg ctgaggcaat aagagaagtt 5220
   agtttgaatg agaattttac tataaatgtt acaagattca atagaccaca aataaatgga 5280
   aaaacaaata gtacattagg tgactttact aatttacttt tacttgaaat aaataattct 5340
   aagcatgaaa aaatattaga caggtttaga gaaattcaag gtcaattaat agaagattta 5400
   agtcatgagt tattttctgg aatagaaatg caaaaggagt taagaaaaat agaaaaagac 5460
   aatctagtat taatgcctat agtatttaca agtggtatag gaataaattc atgggatgat 5520
   gatgaaagat taggaaaaat agtatatgga ttaagtcaaa ctcctcaagt atttttagat 5580
   aatcaggtgt ttgtatataa tgatggtttg aaaatttatt gggatagtat tgatgagatt 5640
   ttgggtgaag ataaagtaga tttgatgttc aaaaaatttg tgatattttt aactgaaata 5700
   gctgatggct cttttaataa agaaagtacc attgctaaga aacgagaata tacggattat 5760
   attttctcaa atgaagatat tgagaaacaa gaaaaagagg cgattaaaaa tgatgttgtt 5820
   gaaataaact atatagagca agatatgaaa aatatttggg aaagcatact tgatatttcg 5880
   attgagaatt atgattgtaa attttttgaa gcgggtggag attctttgag agcgattcaa 5940
   cttagtaata agattcaaga aatgttttca gtaaatgtag atttattgga aatattcaag 6000
   aacccttcta ttagagaaat aagtctatta gttagtaaag aaaaagaaaa tattatagaa 6060
   gggagtttat gatgaaaaaa g 6081
<210> 2
   <211> 833
   <212> DNA
   <213> Streptococcus equi
<400> 2
   aagtgatgaa agtaagtttg atttaacaga tgtccagtac tcctatttaa tcggaagaga      60
   agatgatcag attttaggtg gcgtgggttg ccatgcatat cttgaaatag atggagaaaa     120
   tattgatgag gataagttaa aagaggcttg gaataagctt caatacagac atcccatgct     180
   tagaacaaaa tttacgaaag acgggaagca ggaaatatta tacaaaccgt acagtgaaga     240
   aatagaagtt tttgatttat ctgatcttga tgaagaaacg ctgcatctaa aattagtaga     300
   aattagagaa caaaaatctc ataggaaatt aaatgtaaat caaggtcagg ttgcaggagt     360
   agcactagca aaattttcag atgagaagtc aaggatattt tttgacgtag atttgcttgt     420
   atccgatgta atgagcatga gtattatgat taaagaatta gctgaacttt attcaggagt     480
   agaacttgat aatttgaatg agtatacgtt taaggattat atgcaaaacg gaattggcga     540
   atcaatcaat gatgcagata aggagttttg ggaacaaaaa ataaattcct ttgaaataga     600
   aagaccgaat ttaccattaa ggaaacagcc ggaacaaatt aaagaaacga agtttacaag     660
   aagaaagaga attattaaaa aaagtgaatg ggaaaccata aaagatatag cagcatcgta     720
   tcgaagtaca ccatctatgg ttcttctaac tgcctatgct cttgttcttg aaagatggtg     780
   taatcaggat aaatttttta tcaatatacc gctatttaat agagatttag aaa            833
<210> 3
   <211> 549
   <212> DNA
   <213> Streptococcus equi
<400> 3
   gggttgccat gcatatcttg aaatagatgg agaaaatatt gatgaggata agttaaaaga      60
   ggcttggaat aagcttcaat acagacatcc catgcttaga acaaaattta cgaaagacgg     120
   gaagcaggaa atattataca aaccgtacag tgaagaaata gaagtttttg atttatctga 180
   tcttgatgaa gaaacgctgc atctaaaatt agtagaaatt agagaacaaa aatctcatag 240
   gaaattaaat gtaaatcaag gtcaggttgc aggagtagca ctagcaaaat tttcagatga 300
   gaagtcaagg atattttttg acgtagattt gcttgtatcc gatgtaatga gcatgagtat 360
   tatgattaaa gaattagctg aactttattc aggagtagaa cttgataatt tgaatgagta 420
   tacgtttaag gattatatgc aaaacggaat tggcgaatca atcaatgatg cagataagga 480
   gttttgggaa caaaaaataa attcctttga aatagaaaga ccgaatttac cattaaggaa 540
   acagccgga 549
<210> 4
   <211> 130
   <212> DNA
   <213>
<400> 4
   aagatatagc agcatcgtat cgaagtacac catctatggt tcttctaact gcctatgctc 60
   ttgttcttga aagatggtgt aatcaggata aattttttat caatataccg ctatttaata 120
   gagatttaga 130
<210> 5
   <211> 20
   <212> DNA
   <213> Streptococcus equi
<400> 5
   gggttgccat gcatatcttg 20
<210> 6
   <211> 20
   <212> DNA
   <213> Streptococcus equi
<400> 6
   TCCGGCTGTT TCCTTAATGG 20
<210> 7
   <211> 22
   <212> DNA
   <213> Streptococcus equi
<400> 7
   aagatatagc agcatcgtat cg 22
<210> 8
   <211> 30
   <212> DNA
   <213> Streptococcus equi
<400> 8
   TCTAAATCTC TATTAAATAG CGGTATATTG 30
<210> 9
   <211> 26
   <212> DNA
   <213> Streptococcus equi
<400> 9
   tctatggttc ttctaactgc ctatgc 26

## Claims

1. A method which is:
(a) a method for detecting the presence or absence of *Streptococcus equi* in a sample, the method comprising the step of assessing the presence or absence of the S. *equi eqbE* gene sequence in the sample, or;
(b) a method of diagnosing or prognosing strangles in an equine or camelid mammal, or identifying the mammal as a carrier of strangles, which method comprises the step of assessing the presence or absence of the *S. equi eqbE* gene sequence in a sample from said mammal,
wherein the *S*. *equi eqbE* gene sequence is shown in SEQ ID No 1.

2. A method as claimed in claim 1 wherein the sample is a nucleic acid containing sample obtained from a nasal swab or washes; pus from an abscess; lavages of the guttural pouch.

3. A method as claimed in claim 1 or claim 2 which comprises
(a) the step of assessing the presence or absence of an *S. equi eqbE* signature sequence (SEQ ID No 2), or;
(b) the steps of:
(i) providing a sample of nucleic acid from the mammal, and
(ii) establishing the presence or absence of SEQ ID No 2,
(iii) correlating the presence or absence of SEQ ID No 2, with the presence or absence of *S. equi* in the sample.

4. A method as claimed in claim 3 wherein establishing the presence or absence of SEQ ID No 2 is done by employing a sequence-specific probe which is complementary to a sequence that is present within SEQ ID No 2 or the reverse complement thereof.

5. A method as claimed in claim 3 wherein establishing the presence or absence of SEQ ID No 2 is done by performing a nucleic acid amplification reaction to amplify all or part of SEQ ID No 2 that may be present in the sample.

6. A method as claimed in claim 5 wherein
(i) the nucleic acid amplification reaction is performed by employing two DNA primers to amplify all or part of SEQ ID No 2, and/or
(ii) the amplification reaction yields a copy number of between 50 and 200, and/or
(iii) the nucleic acid amplification reaction is PCR, which is optionally real time PCR, and/or
(iv) the amplification reaction employs one or both of the following primers:
| | | |
|---|---|---|
| eqbE2f: | GGGTTGCCATGCATATCTTG | {Sense} |
| eqbE2r: | TCCGGCTGTTTCCTTAATGG | {Antisense} |
and/or
(v) the amplification reaction employs one or both of the following primers and the following probe that enables the amplification of part of SEQ ID No 2, and specific detection thereof.
| | | |
|---|---|---|
| EqbEf: | AAGATATAGCAGCATCGTATCG | {Sense} |
| EqbEr: | TCTAAATCTCTATTAAATAGCGGTATATTG | {Antisense} |
| Probe: | TCTATGGTTCTTCTAACTGCCTATGC | |

7. A method as claimed in claim 6 wherein
(i) the primers and\or probe are labelled, and/or
(ii) the primers both bind within SEQ ID No 2 or the reverse complement thereof, or one of both primers bind to SEQ ID No 1 or the reverse complement thereof and flank SEQ ID No 2 such that some or all of SEQ ID No 2 is amplified, and/or
(iii) the amplified region which the primers flank is less than 600, 500, 400, 300 nucleotides, more preferably less than 250 nucleotides, more preferably 20 to 200, or 50 to 180, or 100 to 150 nucleotides in length.

8. A method as claimed in any one of claims 3 to 7 wherein the presence of *S. equi* in the sample is confirmed by nucleotide sequencing of nucleic acid present in the sample and\or culturing the sample.

9. A pair of oligonucleotide primers for the amplification of nucleic acid from *Streptococcus equi* but not from *Streptococcus zooepidemicus,*
wherein said pair of primers enables the PCR amplification of some or all of SEQ ID NO 2
wherein, optionally, both primers bind within SEQ ID No 2 or the reverse complement thereof, or one of both primers bind to SEQ ID No 1 or the reverse complement thereof and flank SEQ ID No 2 such that some or all of SEQ ID No 2 is amplified.

10. A pair of primers as claimed in claim 9 wherein both primers bind to SEQ ID No 2 or the reverse complement thereof.

11. A pair of primers as claimed in claim 9 or claim 10 wherein the primers are adapted to amplify 833, or more than 800, 700, 600, 500, 400, 300, 200, 150, 100, 90, 80, 70, 60, 50, 40, 30, or 20 contiguous nucleotides of SEQ ID No 2, and/or
wherein one or both primers are selected from the group consisting of:
| | | |
|---|---|---|
| eqbE2f: | GGGTTGCCATGCATATCTTG | {Sense} |
| eqbE2r: | TCCGGCTGTTTCCTTAATGG | {Antisense} |
| EqbEf: | AAGATATAGCAGCATCGTATCG | {Sense} |
| EqbEr: | TCTAAATCTCTATTAAATAGCGGTATATTG | {Antisense} |

12. An oligonucleotide probe for the detection and identification of nucleic acid from *Streptococcus equi* but not from *Streptococcus zooepidemicus,*
wherein said probe hybridizes under sequence-specific hybridization conditions to SEQ ID No 2 or to the amplification product of a pair primers of claim 10 or 11,
wherein the probe optionally has the sequence:
TCTATGGTTCTTCTAACTGCCTATGC

13. A set of oligonucleotides for the amplification, detection, and identification of nucleic acid from *Streptococcus equi* but not from *Streptococcus zooepidemicus,*
wherein said set comprises:
(a) a pair of primers as defined in any one of claims 9 to 11;
(b) an oligonucleotide probe as defined in claim 12.

14. A kit for use in a method of any one of claims 1 to 8 comprising
(a) a pair of primers as defined in any one of claims 9 to 11;
plus optionally one or more of:
(b) an oligonucleotide probe as defined in claim 12;
(c) instructions for use of the primers in a PCR method for the detection of *S. equi;*
(d) a polymerase, nucleotides, and\or buffer solution;
(e) means for providing the test sample.

15. A method for identifying a *Streptococcus* bacterium in a sample as *Streptococcus equi* comprising use of a pair of primers, probe, or kit as defined in any one of claims 9 to 14.

16. A method, pair of primers, probe, or kit as defined in any one of claims 1 to 15 wherein the mammal is an equine mammal, and optionally wherein the equine mammal is a horse.

## Patentansprüche

1. Verfahren, das:
(a) ein Verfahren zum Nachweis der Gegenwart oder Abwesenheit von *Strep-tococcus equi* in einer Probe ist, wobei das Verfahren den Schritt des Bestimmens der Gegenwart oder Abwesenheit der *S.-equi-eqbE-Gensequenz* in der Probe umfasst; oder
(b) ein Verfahren zur Diagnose oder Prognose von Druse bei einem pferde- oder kamelartigen Säugetier oder zum Identifizieren des Säugetiers als Träger von Druse ist, wobei das Verfahren den Schritt des Bestimmens der Gegenwart oder Abwesenheit der *S.-equi-eqbE-Gensequenz* in der Probe aus dem Säugetier umfasst, worin die *S.-equi-eqbE-Gensequenz* in Seq.-ID Nr. 1 angeführt ist.

2. Verfahren nach Anspruch 1, worin die Probe eine Nucleinsäure enthaltende Probe ist, die von einem Nasenabstrich oder Nasenspülungen; von Eiter aus einem Abszess; Spülungen des Luftsacks stammt.

3. Verfahren nach Anspruch 1 oder Anspruch 2, das Folgendes umfasst:
(a) den Schritt des Bestimmens der Gegenwart oder Abwesenheit einer S.-*equi*-*eqbE*-Signatursequenz (Seq.-ID Nr. 2); oder
(b) die folgenden Schritte:
(i) das Bereitstellen einer Probe von Nucleinsäure aus dem Säugetier und
(ii) das Feststellen der Gegenwart oder Abwesenheit von Seq.-ID Nr. 2,
(iii) das Korrelieren der Gegenwart oder Abwesenheit von Seq.-ID Nr. 2 mit der Gegenwart oder Abwesenheit von *S. equi* in der Probe.

4. Verfahren nach Anspruch 3, worin das Feststellen der Gegenwart oder Abwesenheit von Seq.-ID Nr. 2 durch das Einsetzen einer sequenzspezifischen Sonde erfolgt, die zu einer Sequenz komplementär ist, die innerhalb von Seq.-ID Nr. 2 oder deren reversem Komplement vorliegt.

5. Verfahren nach Anspruch 3, worin das Feststellen der Gegenwart oder Abwesenheit von Seq.-ID Nr. 2 durch das Durchführen einer Nucleinsäureamplifikationsreaktion zum Amplifizieren der gesamten oder eines Teils von Seq.-ID Nr. 2, die in der Probe möglicherweise vorliegt, erfolgt.

6. Verfahren nach Anspruch 5, worin:
(i) die Nucleinsäureamplifikationsreaktion durch das Einsetzen zweier DNA-Primer zum Amplifizieren der gesamten oder eines Teils von Seq.-ID Nr. 2 erfolgt und/oder
(ii) die Amplifikationsreaktion eine Kopienzahl zwischen 50 und 200 hervorbringt und/oder
(iii) die Nucleinsäureamplifikationsreaktion PCR ist, wobei es sich gegebenenfalls um Echtzeit-PCR handelt, und/oder
(iv) die Amplifikationsreaktion mit einem oder beiden der folgenden Primer arbeitet:
| | | |
|---|---|---|
| eqbE2f: | GGGTTGGCCATGCATATCTTG | {Sense} |
| eqbE2r: | TCCGGCTGTTTCCTTAATGG | {Antisense} |
und/oder
(v) in der Amplifikationsreaktion einer oder beide der folgenden Primer und die folgende Sonde eingesetzt werden, die die Amplifikation eines Teils von Seq.-ID Nr. 2 und die spezifische Detektion davon ermöglicht:
| | | |
|---|---|---|
| EqbEf: | AAGATATAGCAGCATCGTATCG | {Sense} |
| EqbEr: | TCTAAATCTCTATTAAATAGCGGTATATTG | {Antisense} |
| Sonde: | TCTATGGTTCTTCTAACTGCCTATGC | |

7. Verfahren nach Anspruch 6, worin:
(i) die Primer und/oder die Sonde markiert sind und/oder
(ii) die Primer beide innerhalb von Seq.-ID Nr. 2 oder dem reversen Komplement davon binden oder einer oder beide Primer an Seq.-ID Nr. 1 oder das reverse Komplement davon binden und Seq.-ID Nr. 2 so flankieren, dass manches oder alles von Seq.-ID Nr. 2 amplifiziert wird, und/oder
(iii) die amplifizierte Region, die die Primer flankieren, weniger als 600, 500, 400, 300 Nucleotide, noch bevorzugter weniger als 250 Nucleotide, noch bevorzugter 20 bis 200 oder 50 bis 180 oder 100 bis 150 Nucleotide, lang ist.

8. Verfahren nach einem der Ansprüche 3 bis 7, worin die Gegenwart von *S. equi* in der Probe durch Nucleotidsequenzierung von in der Probe vorhandener Nucleinsäure und/oder durch Kultivieren der Probe bestätigt wird.

9. Oligonucleotidprimerpaar zur Amplifikation von Nucleinsäure aus *Streptococcus equi,* nicht aber aus *Streptococcus zooepidemicus,*
worin das Primerpaar die PCR-Amplifikation eines Teils oder der gesamten Seq.-ID Nr. 2 ermöglicht,
worin gegebenenfalls beide Primer innerhalb von Seq.-ID Nr. 2 oder dem reversen Komplement davon binden oder einer oder beide Primer an Seq.-ID Nr. 1 oder das reverse Komplement davon binden und Seq.-ID Nr. 2 so flankieren, dass manches oder alles von Seq.-ID Nr. 2 amplifiziert wird.

10. Primerpaar nach Anspruch 9, worin gegebenenfalls beide Primer an Seq.-ID Nr. 2 oder das reverse Komplement davon binden.

11. Primerpaar nach Anspruch 9 oder Anspruch 10, worin die Primer so adaptiert sind, dass sie 833 oder mehr als 800, 700, 600, 500, 400, 300, 200, 150, 100, 90, 80, 70, 60, 50, 40, 30 oder 20 zusammenhängende Nucleotide von Seq.-ID Nr. 2 amplifizieren, und/oder
worin einer oder beide Primer aus der aus Folgendem bestehenden Gruppe ausgewählt sind:
| | | |
|---|---|---|
| eqbE2f: | GGGTTGGCCATGCATATCTTG | {Sense} |
| eqbE2r: | TCCGGCTGTTTCCTTAATGG | {Antisense} |
| EqbEf: | AAGATATAGCAGCATCGTATCG | {Sense} |
| EqbEr: | TCTAAATCTCTATTAAATAGCGGTATATTG | {Antisense} |

12. Oligonucleotidsonde zur Detektion und Identifikation von Nucleinsäure aus *Streptococcus equi,* nicht aber aus *Streptococcus zooepidemicus,*
worin die Sonde unter sequenzspezifischen Hybridisierungsbedingungen an Seq.-ID Nr. 2 oder an das Amplifikationsprodukt eines Primerpaares nach Anspruch 10 oder 11 hybridisiert,
worin die Sonde gegebenenfalls die Sequenz TCTATGGTTCTTCTAACTGCCTATGC aufweist.

13. Oligonucleotidsatz zur Amplifikation, Detektion und Identifikation von Nucleinsäure aus *Streptococcus equi,* nicht aber aus *Streptococcus zooepidemicus,*
worin der Satz Folgendes umfasst:
(a) ein Primerpaar nach einem der Ansprüche 9 bis 11;
(b) eine Oligonucleotidsonde nach Anspruch 12.

14. Set zur Verwendung in einem Verfahren nach einem der Ansprüche 1 bis 8, das Folgendes umfasst:
(a) ein Primerpaar nach einem der Ansprüche 9 bis 11;
zuzüglich gegebenenfalls eines oder mehrerer der Folgenden:
(b) eine Oligonucleotidsonde nach Anspruch 12;
(c) Anweisungen für die Verwendung der Primer in einem PCR-Verfahren zur Detektion von *S*. *equi;*
(d) eine Polymerase, Nucleotide und/oder eine Pufferlösung;
(e) Mittel zur Bereitstellung der Testprobe.

15. Verfahren zur Identifikation eines *Streptococcus-Bakteriums* in einer Probe als *Streptococcus equi,* das die Verwendung eines Primerpaares, einer Sonde oder eines Sets nach einem der Ansprüche 9 bis 14 umfasst.

16. Verfahren, Primerpaar, Sonde oder Set nach einem der Ansprüche 1 bis 15, worin das Säugetier ein pferdeartiges Säugetier ist und worin das pferdeartige Säugetier gegebenenfalls ein Pferd ist.

## Revendications

1. Procédé qui est :
(a) un procédé pour détecter la présence ou l'absence de *Streptococcus equi* dans un échantillon, le procédé comprenant l'étape d'évaluation de la présence ou de l'absence de la séquence du gène *eqbE* de *S*. *equi* dans l'échantillon, ou ;
(b) un procédé pour établir le diagnostic ou le pronostic de la gourme chez un mammifère équin ou camélidé, ou identifier le mammifère comme porteur de la gourme, lequel procédé comprend l'étape d'évaluation de la présence ou de l'absence de la séquence du gène *eqbE* de *S*. *equi* dans un échantillon issu dudit mammifère,
où la séquence du gène *eqbE* de *S*. *equi* est montrée dans SEQ ID NO: 1.

2. Procédé selon la revendication 1, dans lequel l'échantillon est un échantillon contenant un acide nucléique obtenu à partir d'un écouvillonnage ou de lavages nasaux ; de pus provenant d'un abcès ; de lavages de la poche gutturale.

3. Procédé selon la revendication 1 ou la revendication 2, qui comprend
(a) l'étape d'évaluation de la présence ou de l'absence d'une séquence de signature *eqbE* de *S. equi* (SEQ ID NO: 2), ou ;
(b) les étapes de :
(i) mise à disposition d'un échantillon d'acide nucléique issu du mammifère, et
(ii) établissement de la présence ou de l'absence de SEQ ID NO: 2,
(iii) corrélation de la présence ou de l'absence de SEQ ID NO: 2 avec la présence ou l'absence de *S*. *equi* dans l'échantillon.

4. Procédé selon la revendication 3, dans lequel l'établissement de la présence ou de l'absence de SEQ ID NO: 2 est réalisé en employant une sonde spécifique d'une séquence qui est complémentaire à une séquence qui est présente au sein de SEQ ID NO: 2 ou du complément inverse de celle-ci.

5. Procédé selon la revendication 3, dans lequel l'établissement de la présence ou de l'absence de SEQ ID NO: 2 est réalisé en effectuant une réaction d'amplification d'acide nucléique afin d'amplifier l'intégralité ou une partie de SEQ ID NO: 2 qui peut être présente dans l'échantillon.

6. Procédé selon la revendication 5, dans lequel
(i) la réaction d'amplification d'acide nucléique est réalisée en employant deux amorces d'ADN afin d'amplifier l'intégralité ou une partie de SEQ ID NO: 2, et/ou
(ii) la réaction d'amplification génère un nombre de copies compris entre 50 et 200, et/ou
(iii) la réaction d'amplification d'acide nucléique est une PCR, qui est facultativement une PCR en temps réel, et/ou
(iv) la réaction d'amplification emploie l'une ou les deux amorces suivantes :
eqbE2f : GGGTTGCCATGCATATCTTG {Sens}
eqbE2r : TCCGGCTGTTTCCTTAATGG {Antisens} et/ou
(v) la réaction d'amplification emploie l'une ou les deux amorces suivantes et la sonde suivante qui permet l'amplification d'une partie de SEQ ID NO: 2, et la détection spécifique de celle-ci.
| | | |
|---|---|---|
| EqbEf : | AAGATATAGCAGCATCGTATCG | {Sens} |
| EqbEr : | TCTAAATCTCTATTAAATAGCGGTATATTG | {Antisens} |
| Sonde : | TCTATGGTTCTTCTAACTGCCTATGC | |

7. Procédé selon la revendication 6, dans lequel
(i) les amorces et/ou la sonde sont marquées, et/ou
(ii) les amorces se lient toutes deux au sein de SEQ ID NO: 2 ou du complément inverse de celle-ci, ou l'une des deux amorces se lie à SEQ ID NO: 1 ou au complément inverse de celle-ci et flanque SEQ ID NO: 2 de sorte qu'une partie ou l'intégralité de SEQ ID NO: 2 soit amplifiée, et/ou
(iii) la région amplifiée qui est flanquée par les amorces a une longueur inférieure à 600, 500, 400, 300 nucléotides, de manière davantage préférée inférieure à 250 nucléotides, de manière davantage préférée de 20 à 200, ou de 50 à 180, ou de 100 à 150 nucléotides.

8. Procédé selon l'une quelconque des revendications 3 à 7, dans lequel la présence de *S. equi* dans l'échantillon est confirmée par un séquençage de nucléotides de l'acide nucléique présent dans l'échantillon et/ou une mise en culture de l'échantillon.

9. Paire d'amorces oligonucléotidiques pour l'amplification d'un acide nucléique de *Streptococcus equi* mais pas de *Streptococcus zooepidemicus,*
où ladite paire d'amorces permet l'amplification par PCR d'une partie ou de l'intégralité de SEQ ID NO: 2
où, facultativement, les deux amorces se lient au sein de SEQ ID NO: 2 ou du complément inverse de celle-ci, ou l'une des deux amorces se lie à SEQ ID NO: 1 ou au complément inverse de celle-ci, et flanque SEQ ID NO: 2 de sorte qu'une partie ou l'intégralité de SEQ ID NO: 2 soit amplifiée.

10. Paire d'amorces selon la revendication 9, où les deux amorces se lient à SEQ ID NO: 2 ou au complément inverse de celle-ci.

11. Paire d'amorces selon la revendication 9 ou la revendication 10, où les amorces sont adaptées pour amplifier 833, ou plus de 800, 700, 600, 500, 400, 300, 200, 150, 100, 90, 80, 70, 60, 50, 40, 30, ou 20 nucléotides contigus de SEQ ID NO: 2, et/ou
où l'une ou les deux amorces sont sélectionnées dans le groupe consistant en :
| | | |
|---|---|---|
| eqbE2f : | GGGTTGCCATGCATATCTTG | {Sens} |
| eqbE2r : | TCCGGCTGTTTCCTTAATGG | {Antisens} |
| EqbEf : | AAGATATAGCAGCATCGTATCG | {Sens} |
| EqbEr : | TCTAAATCTCTATTAAATAGCGGTATATTG | {Antisens} |

12. Sonde oligonucléotidique pour la détection et l'identification d'un acide nucléique de *Streptococcus equi* mais pas de *Streptococcus zooepidemicus,*
où ladite sonde s'hybride, dans des conditions d'hybridation spécifique d'une séquence, à SEQ ID NO: 2 ou au produit d'amplification d'une paire d'amorces selon la revendication 10 ou 11,
où la sonde possède facultativement la séquence :
TCTATGGTTCTTCTAACTGCCTATGC

13. Jeu d'oligonucléotides pour l'amplification, la détection, et l'identification d'un acide nucléique de *Streptococcus equi* mais pas de *Streptococcus zooepidemicus,*
où ledit jeu comprend :
(a) une paire d'amorces telle que définie dans l'une quelconque des revendications 9 à 11 ;
(b) une sonde oligonucléotidique telle que définie dans la revendication 12.

14. Kit destiné à être utilisé dans un procédé selon l'une quelconque des revendications 1 à 8, comprenant
(a) une paire d'amorces telle que définie dans l'une quelconque des revendications 9 à 11 ;
plus facultativement l'un ou plusieurs de :
(b) une sonde oligonucléotidique telle que définie dans la revendication 12 ;
(c) des instructions pour utiliser les amorces dans un procédé de PCR pour la détection de *S. equi ;*
(d) une polymérase, des nucléotides, et/ou une solution tampon ;
(e) un moyen pour mettre à disposition l'échantillon de test.

15. Procédé pour identifier une bactérie *Streptococcus* dans un échantillon en tant que *Streptococcus equi,* comprenant l'utilisation d'une paire d'amorces, d'une sonde, ou d'un kit tel que défini dans l'une quelconque des revendications 9 à 14.

16. Procédé, paire d'amorces, sonde, ou kit tel que défini dans l'une quelconque des revendications 1 à 15, où le mammifère est un mammifère équin, et facultativement où le mammifère équin est un cheval.
